(19) Europäisches Patentamt
European Patent Office
Office européen des brevets

(11) EP 4 257 041 A1

(12) EUROPEAN PATENT APPLICATION
published in accordance with Art. 153(4) EPC

(43) Date of publication:
11.10.2023 Bulletin 2023/41

(51) International Patent Classification (IPC):
A61B 5/107 (2006.01)

(21) Application number: 21902349.6

(52) Cooperative Patent Classification (CPC):
A61B 5/00; A61B 5/08; A61B 5/107

(22) Date of filing: 17.11.2021

(86) International application number:
PCT/CN2021/131170

(87) International publication number:
WO 2022/121642 (16.06.2022 Gazette 2022/24)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 07.12.2020 CN 202011427775
02.02.2021 CN 202110143261

(71) Applicant: Shenzhen Lifetech Respiration
Scientific Co., Ltd.
Shenzhen, Guangdong 518000 (CN)

(72) Inventors:
• CHEN, Quan
Shenzhen, Guangdong 518000 (CN)
• LI, AnNing
Shenzhen, Guangdong 518000 (CN)

(74) Representative: Prinz & Partner mbB
Patent- und Rechtsanwälte
Rundfunkplatz 2
80335 München (DE)

(54) **MEASURING DEVICE**

(57) A measuring device includes a hollow catheter and a guide wire which is insertable into the hollow catheter, where a catheter developing structure is arranged on the catheter, a distance of the catheter developing structure from a distal end of the catheter being less than a length of diseased tissue; a guide wire developing structure is arranged on a distal end of the guide wire, and a scale mark zone is arranged on the guide wire (2), a proximal end of the catheter can enter into the scale mark zone; the scale mark zone is configured to directly or indirectly measure a distance between the proximal end of the catheter and the end portion of the guide wire, and further measure the length of the diseased tissue.

Fig. 1

EP 4 257 041 A1

**Description**

**Technical Field**

The embodiments relate to the field of medical instruments and to a measuring device.

**Background**

Currently, an updated treatment for emphysema is to implant an elastic coil (such as a lung volume reduction elastic coil) as an implant into the location of human lung lesions. The lung volume reduction elastic coil is designed and made of nickel-titanium memory alloy metal wire, which is curved in its natural state. The lung volume reduction elastic coil may elastically deform under the action of external force. For example, the lung volume reduction elastic coil can be in a straight shape under the constraint of the loading system, so as to be implanted into the lung through the bronchoscope working channel. When the lung volume reduction elastic coil is delivered into the bronchus of the emphysema area, the lung volume reduction elastic coil loses the constraint of the loading system and recovers the deformation into a bending shape of a natural shape (that is, the shape when not subjected to an external force). At the same time, the emphysema area is squeezed under the pulling action of the lung volume reduction elastic coil, the gas in the bronchus is discharged, and the volume of the lung tissue in the emphysema area is reduced, so as to enable the peripheral relatively healthy lung tissue to have a better physiological function.

Before implantation, the bronchial length of emphysema area is measured with measuring guide wire, so as to determine the specifications of the lung volume reduction elastic coil.

**Summary**

An object of the embodiments is to provide a measuring device that addresses the need in the art to provide a measuring device for determining the specifications of an implant.

The embodiments may include: a measuring device is provided to measure a length of diseased tissue to determine specifications of an implant. The measuring device includes a catheter and a guide wire. The catheter is a hollow tube structure, where a catheter developing structure is arranged on the catheter, a distance between the catheter developing structure and a distal end portion of the catheter not exceeding an axial length corresponding to minimum specifications of the implant. The guide wire is insertable into the catheter, and a guide wire developing structure is arranged on a distal end of the guide wire; a scale mark zone is arranged on the guide wire, a proximal end portion of the catheter can enter into the scale mark zone, and the scale mark zone is configured to directly or indirectly measure a distance between the catheter developing structure and the guide wire developing structure to determine the specifications of the implant.

In one embodiment, a length of the catheter is a; the scale mark zone is configured to measure a distance between the catheter developing structure and the guide wire developing structure; a plurality of scales are provided in the scale mark zone, one of the plurality of scales disposed at a farthest end in the scale mark zone being a reference mark point, and one of the plurality of scales farthest from the reference mark point being a last mark point; a distance between the reference mark point and the guide wire developing structure is b, and a distance between the reference mark point and the last mark point is f, a≤b+f.

In one embodiment, the catheter developing structure is disposed at the distal end portion of the catheter, and b=a.

In one embodiment, the minimum specifications of the implant correspond to an axial length c, a≥b-c.

In one embodiment, a distance between the reference mark point and a nearest scale is equal to the axial length corresponding to the implant with the minimum specifications.

In one embodiment, a distance between any one of the plurality of scales except the reference mark point and the reference mark point is equal to the axial length of the implant with the same specifications.

In one embodiment, the plurality of scales are equally spaced apart.

In one embodiment, the catheter developing structure is disposed at the distal end portion of the catheter.

In one embodiment, a distance between the proximal end portion of the catheter and the catheter developing structure is a ranging from 900 mm to 950 mm.

In one embodiment, the measuring device measures the diseased tissue; the proximal end portion of the catheter can enter into the scale mark zone in response to the guide wire developing structure being aligned with the distal end of the diseased tissue, and the catheter developing structure being aligned with the proximal end of the diseased tissue.

During measurement with the measuring device provided in the embodiments, the guide wire is inserted into the catheter before being implanted into the lumen channel of the diseased tissue as a whole; the guide wire is pushed to align the guide wire developing structure with the distal end portion of the diseased tissue, and the catheter is pushed to align the catheter developing structure with the proximal end portion of the diseased tissue. In this way, a distance between the guide wire developing structure and the catheter developing structure, directly or indirectly converted by the scale mark, is the length of the diseased tissue, thereby determining specifications of the implant.

**Brief Description of the Drawings**

FIG 1 is schematic perspective view of a measuring device provided in an embodiment;

FIG 2 is a schematic perspective view of a catheter of a measuring device provided in an embodiment;

FIG 3 is a schematic perspective view of a first embodiment of a guide wire of a measuring device provided in an embodiment;

FIG 4 is an enlarged schematic view of portion S in FIG 3;

FIG 5 is a schematic structural diagram of a guide wire of a measuring device provided in an embodiment;

FIG 6 is a schematic perspective view of another embodiment of a guide wire of a measuring device provided in an embodiment;

FIG 7 is an enlarged schematic view of portion G in FIG 5;

FIG 8 is a schematic view of a guide wire reaching a distal end of a bronchus as measured by a measuring device provided in an embodiment;

FIG 9 is an enlarged schematic view of portion A in FIG 8;

FIG 10 is an enlarged schematic view of portion B in FIG 8;

FIG 11 is a schematic view of a catheter reaching a distal end of a working channel of a bronchoscope as measured by a measuring device provided in an embodiment;

FIG 12 is an enlarged schematic view of portion C in FIG 11;

FIG 13 is an enlarged schematic view of portion D in FIG 11;

FIG 14 is a schematic view of an end cover of a catheter of a measuring device being located in a corresponding scale mark zone of a guide wire provided in an embodiment;

FIG 15 is a measuring principle diagram of a measuring device provided in an embodiment;

FIG 16 is a schematic view of a distal end of a catheter reaching and being aligned with a distal end of a guide wire as measured by a measuring device provided in an embodiment;

FIG 17 is an enlarged schematic view of portion E in FIG 16;

FIG 18 is an enlarged schematic view of portion F in FIG 16.

**Detailed Description of the Embodiments**

In order to make the objects, schemes, and advantages of the embodiments clearer, the embodiments will be further illustrated in detail below with reference to the drawings and embodiments. It is understood that the particular embodiments described herein are illustrative only and are not intended as limiting.

It is noted that when an element is referred to as being "fixed" or "arranged" on another element, it can be directly on the other element or an intervening element(s) may be present as well. When an element is referred to as being "connected" to another element, it can be directly connected to the other element or an intervening element(s) may be present as well.

It is further noted that the directional terms left, right, upper, lower, and the like in the embodiment are merely relative concepts to each other or references are made to the normal use of the product, and therefore theses terms should not be considered as limiting.

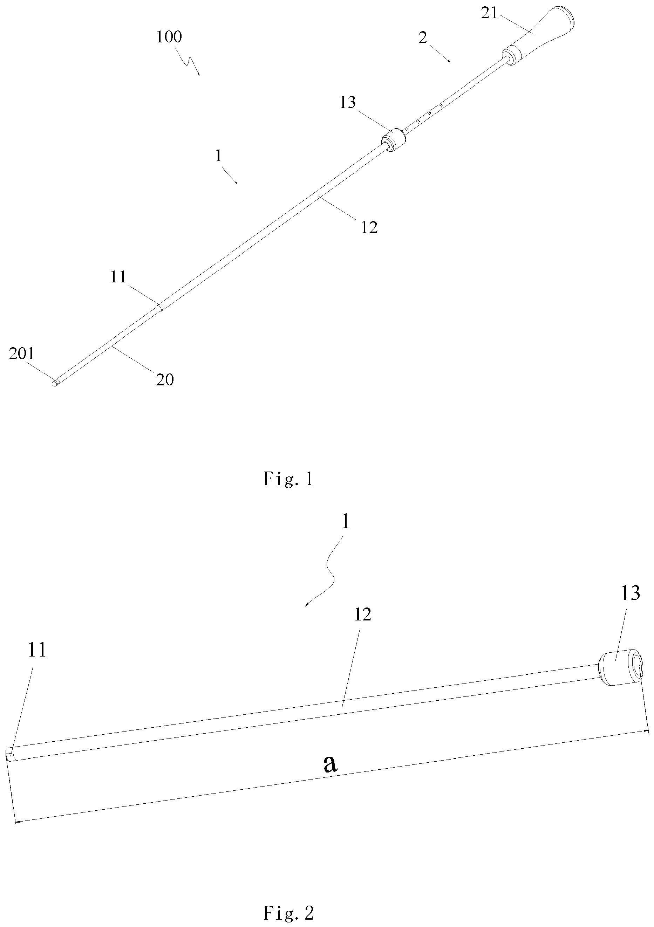

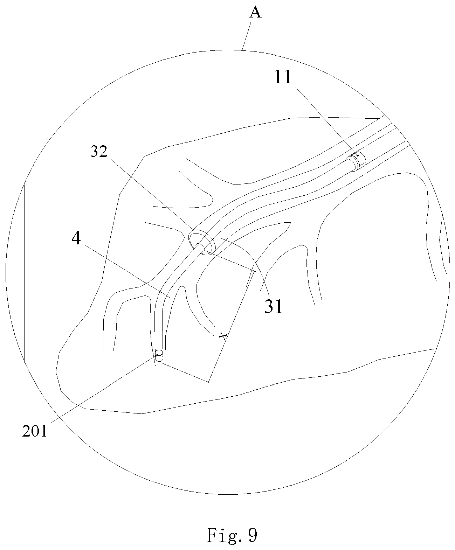

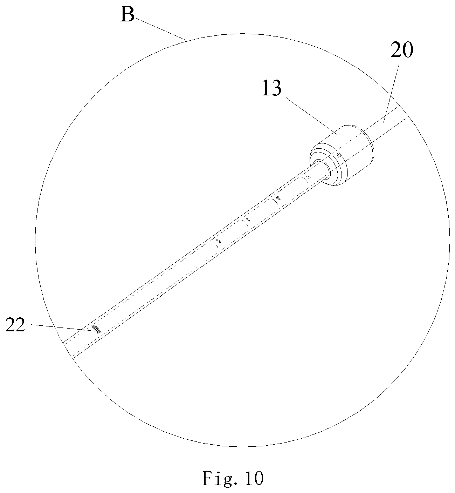

FIGS. 1 to 16 are various relevant schematic diagrams of a measuring device and for measuring a length of diseased tissue using the measuring device provided in an embodiment.

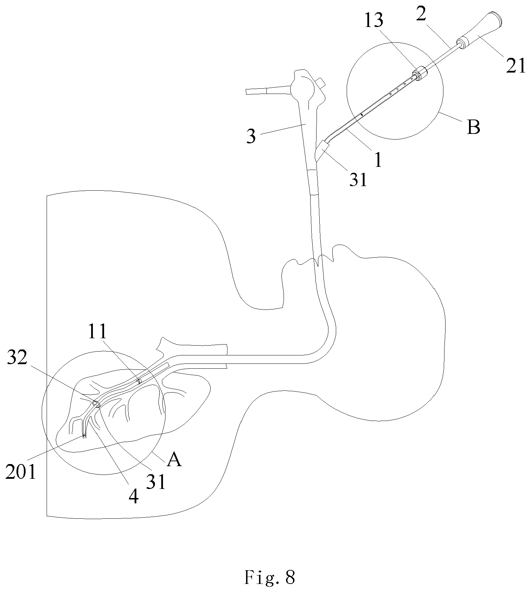

FIG 1 is a relevant schematic diagram of a measuring device 100 according to an embodiment. The measuring device 100 is configured to measure the length of diseased tissue to determine specifications of an implant. For example, the measuring device 100 is used in combination with a medical tube endoscope 3 (the medical tube endoscope 3 is shown in FIG 8, and may be a bronchoscope) and an X-ray developing apparatus (the X-ray developing apparatus is not shown in the drawings, and may be a DSA apparatus) to measure the length of the diseased tissue in the lumen channel (for example, a bronchus), so as to determine specifications of the implant.

The measuring device 100 includes a catheter 1 and a guide wire 2 which is insertable into the catheter 1, where the guide wire 2 is configured to guide the catheter 1 to move forward in the body.

As shown in FIG 2, the catheter 1 is a hollow tube structure. The catheter 1 includes a catheter body 12 at a distal end side thereof, and an end cover 13 provided at the proximal end side of the catheter body 12, the catheter body 12 being fixedly connected to the end cover 13. The catheter body 12 is a hollow tube body with a single-layer or multi-layer structure, which is fixedly connected to the end cover 13 by glue bonding or other manners of bonding. The catheter 1 has good flexibility, self-lubrication, and flexural resistance. To ensure that the catheter 1 can be freely pushed within the bronchoscope, the maximum outer diameter of the catheter body 12 needs to be smaller than the inner diameter of the working channel of the bronchoscope.

The catheter 1 is provided with a catheter developing structure 11, to facilitate the observation of the position of the catheter 1 in the human tissue via an X-ray developing apparatus. In the present embodiment, the catheter developing structure 11 is disposed at the distal end portion of the catheter body 12 (namely, the distal end portion of the catheter 1). As the catheter developing structure 11 is disposed at the distal end portion of the catheter body 12, the position of the catheter developing structure 11 can be observed at the same time when observing the end portion of the catheter body 12, so that the position of the catheter developing structure 11 in the human tissue can be more conveniently observed and the length of the diseased tissue can be more conveniently measured. During measurement, the catheter developing structure 11 is aligned with the proximal end portion of the diseased tissue.

In other embodiments, the catheter developing structure 11 may be provided at a position of a certain length from the distal end portion of the catheter 1, as long as a length of the catheter developing structure 11 and the distal end portion of the catheter 1 does not exceed the axial direction corresponding to a minimum specification of the implant. This prevents the distal end portion of the catheter 1 from colliding with the inner wall of the human tissue before the catheter developing structure 11 reaches the target position to be measured. The implant in the embodiment is a lung volume reduction elastic coil, with a curled natural shape and a straight delivery shape. The axial length, corresponding to the minimum specification in the embodiment, refers to the axial length of the lung volume reduction elastic coil with a minimum specification in the straight delivery shape.

Referring to FIG 2, a length from the proximal end surface of the end cover 13 of the catheter 1 to the catheter developing structure 11 is defined as a, that is, a length of the catheter 1 is a, which is known data after manufacture. In an embodiment, the size of a may range from 900 mm to 950 mm.

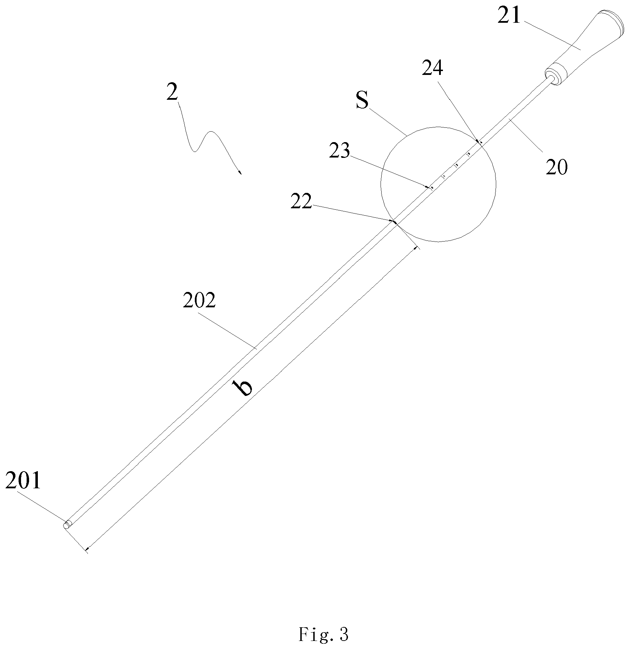

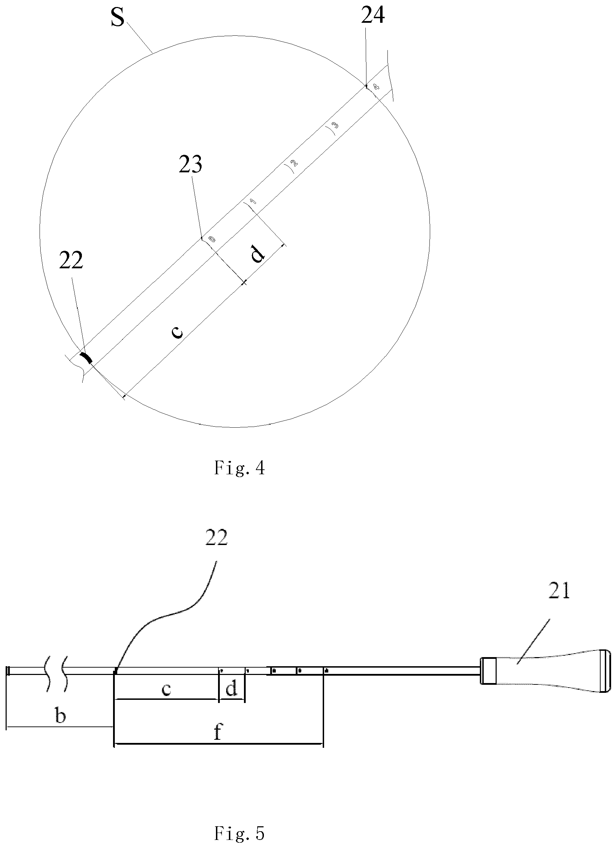

As shown in FIGS. 3 and 4, the guide wire 2 includes a guide wire body 20 at the distal end side thereof and a handle 21 disposed on the proximal end side of the guide wire body 20, the guide wire body 20 being fixedly connected to the handle 21.

The handle 21, with properties of being easily pushed and held, may be made of stainless steel or medical plastic.

The distal end portion of the guide wire body 20 is provided with a guide wire developing structure 201 to facilitate the observation of the position of the guide wire 2 in the human tissue by an X-ray developing apparatus.

During measurement, the guide wire developing structure 201 is aligned with the distal end portion of the diseased tissue. It should be noted that, due to the insufficient machining accuracy in the prior art, machining errors may occur, such as a case where the guide wire developing part 201 is not located at the distal end portion of the guide wire body 20. Therefore, the guide wire developing part 201 should be considered to be disposed on the distal end portion of the guide wire body 20 in response to a distance between the guide wire developing part 201 and the distal end portion of the guide wire body 20 not exceeding 3 mm.

The proximal end side of the guide wire body 20 is provided with a scale mark zone S for directly or indirectly measuring a distance between the catheter developing structure 11 and the end portion of the guide wire developing structure 201.

The guide wire developing structure 201 and the catheter developing structure 11 may be made of X-ray-opaque metal materials, such as gold, platinum, or tantalum.

Referring to FIGS. 3 to 5, when the measuring device 100 is used, the proximal end portion of the catheter 1 can enter into the scale mark zone S in response to the guide wire developing structure 201 being aligned with one end (that is, the distal end) of the diseased tissue and the catheter developing structure 11 being aligned with the other end (that is, the proximal end) of the diseased tissue; and the scale mark zone S is located outside the patient body during measurement.

Since the catheter developing structure 11 in the present embodiment is disposed at the distal end portion of the catheter 1, the measuring device in the present embodiment can calculate a distance between the two developing structures (the guide wire developing structure 201 and the catheter developing structure 11) by subtracting the length of the catheter 1 from a distance between the proximal end portion of the catheter 1 and the guide wire developing structure 201 during measurement, where a distance between the two developing structures is equal to the length of the diseased tissue, thereby selecting an appropriate implant specification for treatment, such as emphysema. It should be noted that since a distance between the position of the scale mark zone S and the guide wire developing structure 201 is known data when the guide wire is manufactured, a distance between the proximal end portion of the catheter 1 and the guide wire developing structure 201 can be obtained according to the reading of the scale mark zone S.

If the catheter developing structure 11 is provided at a position of a certain length from the distal end portion of the catheter 1, since the distance L between the proximal end portion of the catheter and the catheter developing structure 11 is already obtained after the catheter 1 is manufactured (which is not shown in the drawings, and is known data when manufacturing is completed at the factory), the distance between the two developing structures (the guide wire developing structure 201 and the catheter developing structure 11) can be calculated by subtracting this distance L from the distance between the proximal end of the catheter 1 and the guide wire developing structure 201 during measurement.

Taking the measurement of the length of the diseased tissue of a bronchus by the measuring device 100 as an example, the use of the measuring device will be described. The medical tube endoscope 3 is pushed along the trachea to the proximal end portion of the bronchial diseased tissue. For example, the distal end of the bronchoscope 3 is delivered to the subsegmental bronchial opening to be aligned (namely, the proximal end of the diseased site), and the guide wire 2 is inserted into the catheter 1 in vitro to form a component. The component is placed into the working channel of the bronchoscope 3, where keeping the position of the catheter 1 stationary, the guide wire 2 is pushed towards the distal end, so that the guide wire developing structure 201 contact with the bronchial wall (namely, the guide wire developing structure 201 is aligned with the distal end of the diseased tissue); then keeping the position of the guide wire 2 stationary, the catheter 1 is pushed towards the distal end along the guide wire 2, so that the catheter developing structure 11 on the catheter 1 is aligned with the proximal end of the diseased tissue (since the distal end of the bronchoscope 3 is aligned with the proximal end of the diseased tissue, it is only necessary to align the catheter developing structure 11 with the distal end of the bronchoscope 3), the proximal end portion of the catheter 1 enter into the scale mark zone S at this time; and then the distance between the two developing structures is calculated according to the above-mentioned known length data.

Since the measuring device 100 provided in the embodiments does not provide with a plurality of developing structures at equal intervals at the distal end of the guide wire body 20 of the guide wire 2, the problem of measurement failure or measurement inaccuracy can be avoided, which is caused by the interference of the plurality of developing structures when the guide wire is bent. Further, since the number of the developing structures provided in the embodiments is relatively smaller than the prior art, the risk of the metal developing structures falling off into the organ may be greatly reduced, improving the operability and safety of the operation.

In the embodiment, the scale mark zone S is configured to directly or indirectly measure a distance between the catheter developing structure 11 and the guide wire developing structure 201, which is a length of the diseased tissue. A plurality of scales are provided in the scale mark zone S, one of the plurality of scales disposed at a farthest end in the scale mark zone S being a reference mark point 22, and one of the plurality of scales farthest from the reference mark point 22 being a last mark point 24; a distance between the reference mark point 22 and the last mark point 24 is f. The portion of the guide wire body 20 from the distal end portion thereof to the reference mark point 22 is a main section 202, and a guide wire developing structure 201 is provided at the distal end portion of the main section 202, so as to observe the position of the guide wire developing structure 201 and measure the length of the diseased tissue.

The main section 202 may be machined from a metal wire with good elasticity and flexibility, as well as a better deliverability. The deliverability refers to that the main section 202 has desired rigidity to ensure that the guide wire 2 is capable of moving forward along a hollow channel of the catheter 1 when being pushed through the catheter 1, and will not be excessively bent. The guide wire 2 may be bent in the catheter or body tissue caused by the excessive bending and consequently fail to continue moving forward.

The distal end of the main section 202 is made of a biocompatible polymer material, such as silica gel, Teflon, PEBA material or other polymer materials. The manufacturing process can be adjusted to make the main section 202 become harder from the distal end to the proximal end.

In one embodiment, the guide wire developing structure 201 can be arranged at the distal end portion of the main section 202 in an embedded manner. When the guide wire 2 extends into the tissue such as a bronchus in the human body, with the distal end of the main section 202 of the guide wire 2 touching the inner wall of the tissue such as the bronchial wall, the distal end will bend firstly because of being relatively soft, while the proximal end has a small degree of change in bending. The operator can observe the bending degree of the distal end of the main section 202 more directly according to the X-ray developing apparatus, and hence determine whether the guide wire 2 reaches the predetermined position. Likewise, the catheter developing structure 11 may be arranged in this manner.

In the embodiment, a distance between the guide wire developing structure 201 and the reference mark point 22 is b, which is a known value ranging from 850 mm to 1050 mm. The distance between the reference mark point 22 and the distal end of the guide wire developing structure 201 is less than or equal to the length of the catheter 1 (that is, b≤a), and the distance between the last mark point 24 and the distal end portion of the guide wire developing structure 201 is greater than the length of the catheter 1. It should be noted that the catheter 1 and the guide wire 2 in the body may be in a curved state due to the need to adapt to the shape of the lumen channel of the human body. The "length" referred to herein is a straight distance in response to the catheter 1 and the guide wire 2 being in a straight state, and the "length" referred to herein is an arc length in response to the catheter 1 and the guide wire 2 being in a curved arc state. In the embodiments, the guide wire 1 and the catheter 2 can be considered to have the same shape with the former being inserted into the latter.

In another embodiment, the plurality of scales are evenly spaced apart so as to facilitate rapid reading of the values on the scale mark zone S and rapid subsequent evaluation. Further, in order to conveniently read the data of the scale mark zone S outside the human body, the reference mark point 22 and a plurality of scales are provided on the side of the guide wire body 20 near the proximal end, with the scale mark zone S being located outside the patient's body. Additionally, in an embodiment, the scale mark zone S is exposed to the outside of the working channel of the medical tube endoscope during measurement, which is convenient for visual observation.

Referring to FIG 4, the distance between the reference mark point 22 and a first measuring mark point 23 is equal to the axial length c corresponding to the minimum specification of the implant. According to specifications of the implant (such as the lung volume reduction elastic coil) in the prior art and the anatomical and physiological techniques of the lung, c may be a minimum specification of a lung implant, which is determined according to the type of the implant, namely, known data. For example, if specifications of a group of lung volume reduction elastic coils are 90 mm, 110 mm, 130 mm and 150 mm, the size of c may be set as 90 mm, where in one embodiment, the size of c may range from 90 mm to 110 mm. The measuring mark point closest to the reference mark point 22 in the plurality of scales is the first measuring mark point 23. The distance between the first mark point 23 and the reference mark point 22 is minimal, and the distance is equal to the minimum specification of the implant, where the minimum specification may be the minimum axial length of the lung volume reduction elastic coil in a straight state.

The distance between two adjacent scales in the plurality of scales is defined as d, which is also the length difference value between various specifications of implants, where d is determined according to the type of an implant, namely, known data. The distance between any one of the plurality of scales except the reference mark point 22 and the reference mark point 22 is made to be equal to the axial length of an implant instrument with one specification, so that the measuring device 100 can more quickly assist in selecting specifications and model of the implant instrument. For example, if the specifications of a group of lung implants are 90 mm, 110 mm, 130 mm, and 150 mm, the value of d is 20 mm. As an embodiment, the size of d may range from 10 mm to 20 mm. As a specific embodiment, referring to FIGS. 3 and 4, the scales can be set to five, that is, there are four equidistant measurement units between the five scales. A distance between each unit is 10 mm to 20 mm, and a corresponding size reading can be marked beside each scale to facilitate the reading of the size, for example, 0, 1, 2, 3 and 4 can be marked successively from the first measurement mark point 23 to the last mark point 24.

In an embodiment, a, b, c and f satisfy a≥b-c and a≤b+f, so that the specifications of the implant can be quickly determined.

In one embodiment, the catheter developing structure 11 is disposed at the distal end portion of the catheter 1, and b=a.

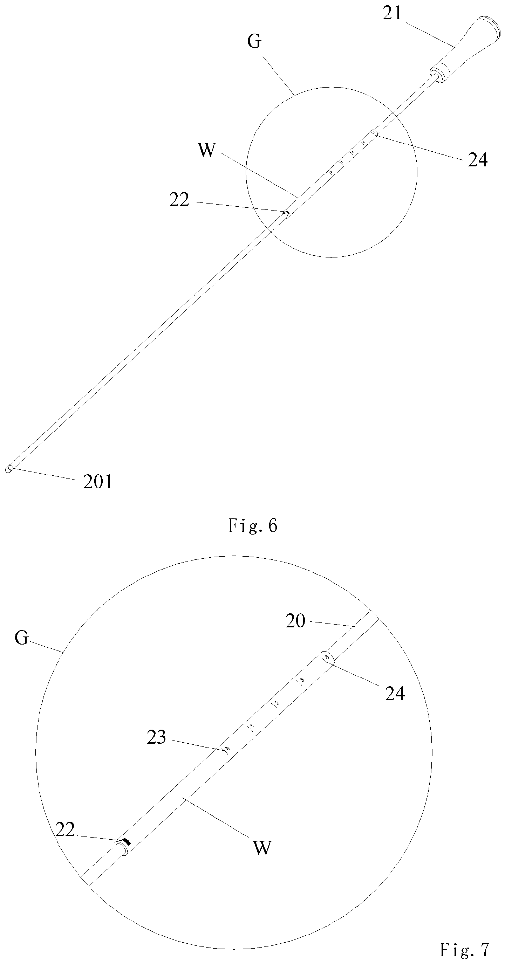

In an embodiment, as shown in FIGS. 5 and 6, the reference mark points 22 and the respective scales on the guide wire body 20 may be arranged by laser marking and engraving directly on the guide wire body 20; or as shown in FIGS. 6 and 7, the reference mark points 22 are marked on the distal end portion of a heat-shrinkable sleeve W, the last mark points 24 are marked on the proximal end portion of the heat-shrinkable sleeve W, and the corresponding respective middle scales are marked on the middle thereof, and then the heat-shrinkable sleeve W is sleeved on the guide wire body 20.

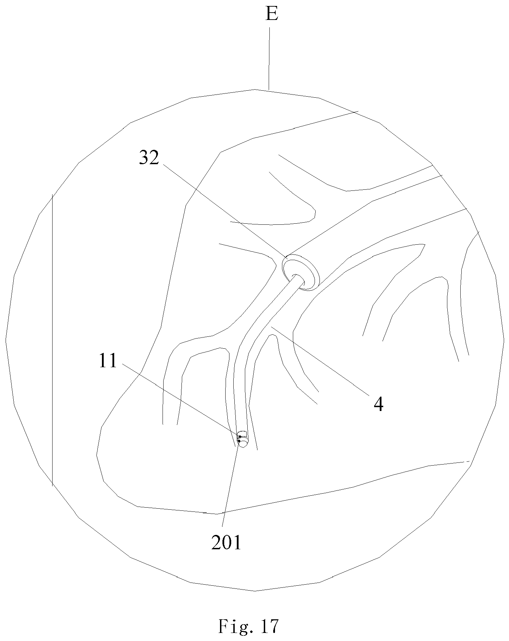

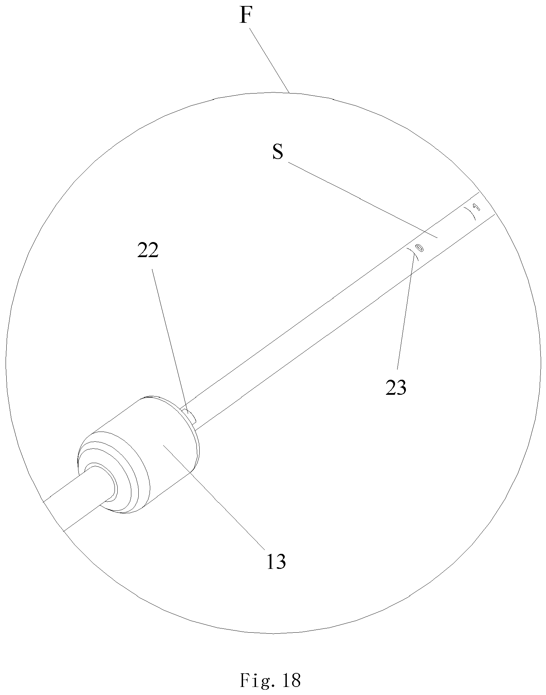

Referring to FIGS. 8 to 18, a method for measuring diseased tissue in a lumen channel is further provided in one embodiment, as well as a medical tube endoscope 3, an X-ray developing apparatus (not shown) and the above-mentioned measuring device, including the following steps:

at step A, as shown in FIGS. 8 to 10, the bronchoscope 3 was deployed to the target lumen channel of the human tissue, and the distal end portion 32 of the medical tube endoscope 3 was aligned with the proximal end of the diseased tissue 4;

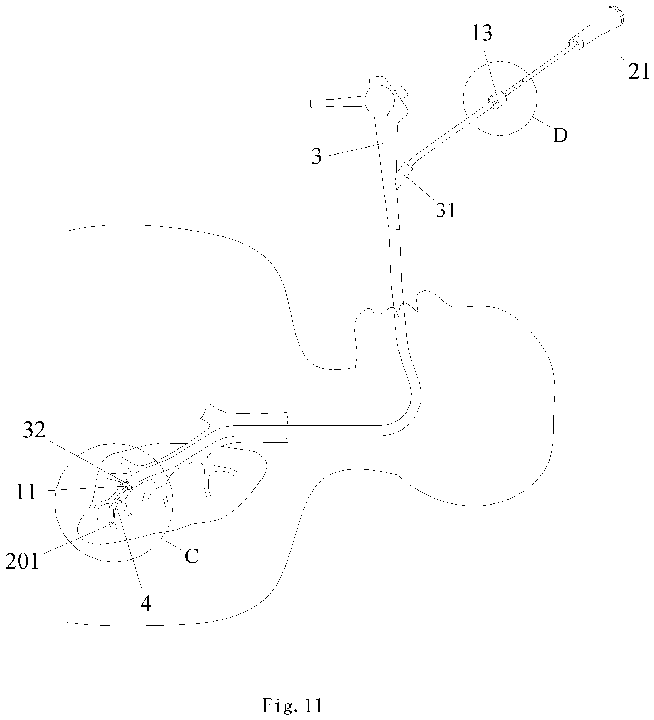

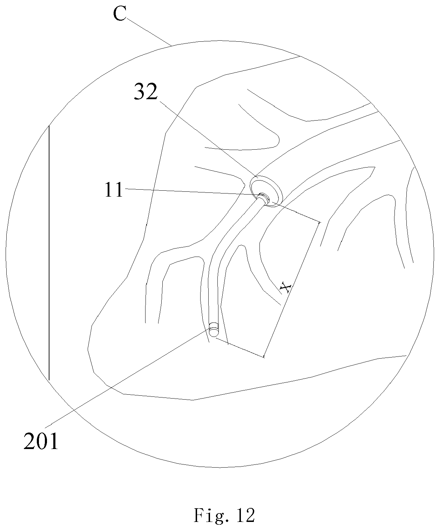

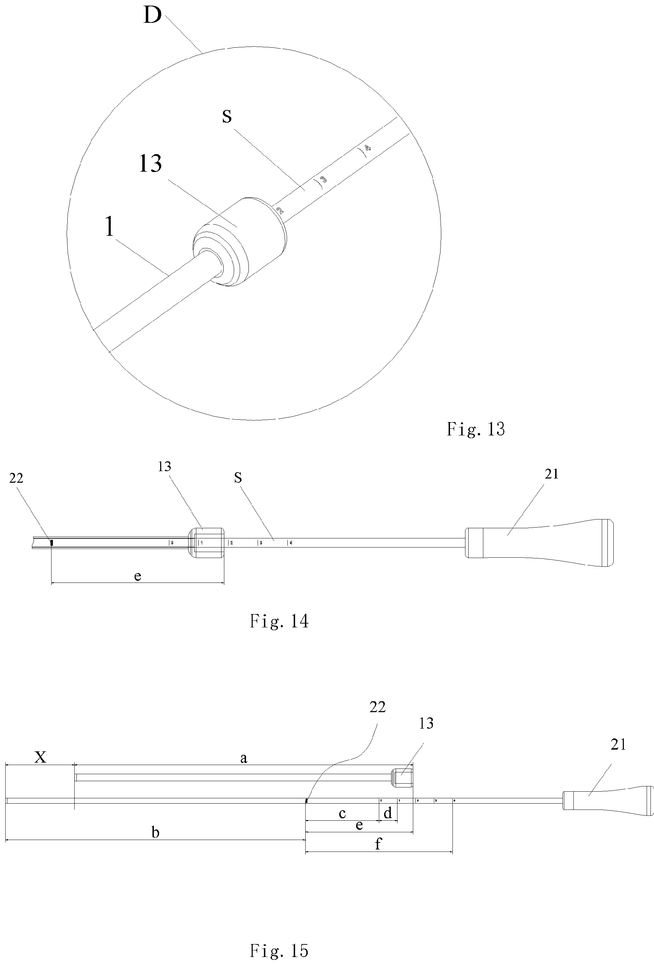

at step B, in vitro, the guide wire 2 was inserted into the hollow inner cavity of the catheter 1 before assembling, and the guide wire 2 of the assembled measuring device was delivered through the working channel 31 of the medical tube endoscope 3 to extend into the airway of the bronchus 4 to be implanted; the guide wire developing structure 201 at the distal end of the guide wire 2 was observed through the X-ray developing apparatus when the guide wire body 20 extended into the human tissue such as the bronchus 4 in the human body; and the delivery of the guide wire 2 was stopped when it was observed through the X-ray developing apparatus that the distal end of the guide wire developing structure 201 had reached the farthest end position of the diseased tissue 4; and

at step C, referring to FIGS. 11 to 15, keeping the position of the guide wire 2 unchanged, the catheter 1 was pushed towards the distal end to observe the position of the catheter developing structure 11 through the X-ray developing apparatus; as shown in FIGS. 11 and 12, the delivery of the catheter 1 was stopped when the catheter developing structure 11 reached and was aligned with the opening of the subsegmental bronchus (namely, reached the distal end portion 32 of the medical tube endoscope 3, namely, reached the nearest end of the diseased tissue 4), the proximal end portion of the catheter 1 being located in the scale mark zone S of the guide wire body 20 at this moment; a distance between the catheter developing structure 11 and the guide wire developing structure 201 was defined as x, which was the length to be measured for the target bronchus 4 diseased tissue, and was also the basis for selecting the specifications of the implant. Referring to FIGS. 12 and 13, the distance from the reference mark point 22 to the end surface of the proximal end portion of the catheter 1 (that is, the proximal end of the end cover 13) is defined as e. The value of e is estimated according to the scale mark zone S corresponding to the proximal end portion of the catheter 1 (that is, the end portion of the end cover 13), and then the value of x is measured. The value of x may be equivalently measured using the following formula:

$$x = e + b - a;$$

The corresponding specification of an implant is selected according to the measured x value. It should be noted that FIG 15 is used to illustrate the axial relative position relationship of the catheter 1 and the guide wire 2 in the body of the patient. To facilitate the display of the scale mark zone S, the catheter 2 is decomposed and radially moved in parallel. In addition, it should also be noted that the interval corresponding to X in FIG 15 is the length of the diseased tissue, which is illustrated in the form of a straight line in the embodiment for the convenience of explanation.

By way of example, assuming that the length specifications of the lung implant are 90 mm, 110 mm, 130 mm and 150 mm, the size c is 90 mm and d is 20 mm in the embodiments. When the size a is equal to the size b, the length x of the target bronchus 4 is equal to the value of e according to the formula. As shown in FIGS. 14 and 15, when the end cover 13 of the catheter 1 is located between markers 1 and 2, the size e is 110 mm to 130 mm, that is, the length of the target bronchus 4 (namely, x) is 110 mm to 130 mm. In order to ensure that the implant will not be at the edge of lung for a long time after implantation (easy to cause pneumothorax), the implant with a length of 110 mm is selected for implantation. According to this method, the implant with a length of 90 mm is selected in response to the end cover 13 being located between markers 0 and 1; the implant with a length of 130 mm is selected in response to the end cover 13 being located between markers 2 and 3; and the implant with a length of 150 mm is selected in response to the end cover 13 being located between markers 3 and 4.

The method for measuring the length of diseased tissue using the above-mentioned measuring device provided in the embodiments, by equivalent length measurement, it can be more convenient and intuitively measured since the scale mark zone S thereof is arranged at the proximal end, which is beneficial to quickly select an implant with a suitable specification. In addition, since it uses relatively fewer developing structures, the problem of inaccurate measurement, caused by mutual interference when the guide wire is bent due to excessive developing structures, can be avoided, further greatly reducing the risk of the metal developing structures falling off into the organ, and improving the operability and safety of the operation.

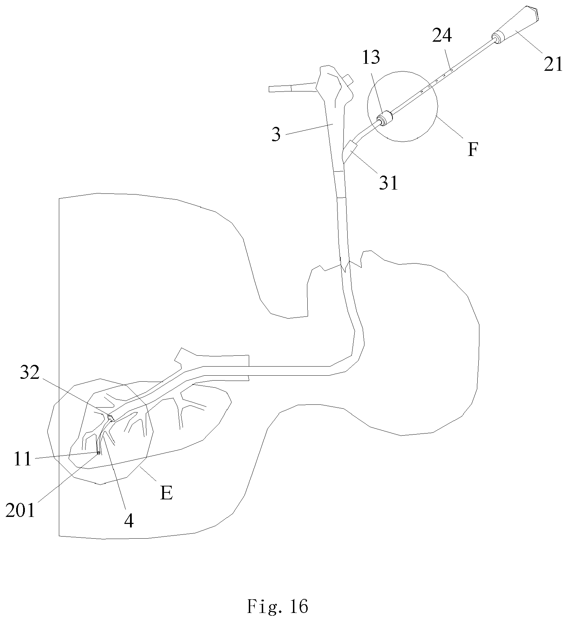

The advantageous effects of the measuring device and measuring method provided in the embodiments also include: referring to FIGS. 16 to 18, after selecting an implant with an appropriate specification according to the value of x, when the catheter 1 is further pushed towards the distal end to serve as the implantation channel of the implant (at this time, keeping the position of the guide wire 2 unchanged), the delivery of the catheter 1 can be stopped (at this time, the catheter 1 reaches the furthest end of the diseased bronchus 4) and the guide wire 2 can be withdrawn when the proximal end (that is, the end cover 13) of the catheter 1 is aligned with the reference mark point 22 of the guide wire 2 through in vitro observation without the aid of X-ray developing apparatus; the implantation of the implant is completed by pushing the implant selected in step d through the hollow lumen of the catheter 1 to the distal end portion of the catheter 1. That is, in the subsequent pushing stage of the catheter 1, the catheter 1 can be observed through in vitro observation without the aid of an X-ray developing apparatus and the pushing of the catheter 1 can be completed, so that the irradiation time of the radiation worker and the subject to X-ray radiation can be effectively shortened, thereby better protecting the health of the worker and the subject.

Described above are merely exemplary embodiments which are not intended to cause any limitation. Any modification, equivalent replacement or improvement made within the spirit and principles of the embodiments shall fall within the scope of the embodiments.

**Claims**

1. A measuring device configured to measure a length of diseased tissue to determine specifications of an implant, the measuring device comprising:

   a catheter having a hollow tube structure; wherein a catheter developing structure is arranged on the catheter, and a distance between the catheter developing structure and a distal end portion of the catheter does not exceed an axial length corresponding to minimum specifications of the implant; and
   a guide wire configured to be inserted into the catheter; wherein a guide wire developing structure is arranged on a distal end of the guide wire; and wherein a scale mark zone is arranged on the guide wire, and a proximal end portion of the catheter can enter into the scale mark zone, the scale mark zone configured to measure a distance between the catheter developing structure and the guide wire developing structure to determine the specifications of the implant.

2. The measuring device according to claim 1, wherein a length of the catheter is a; the scale mark zone is configured to measure a distance between the catheter developing structure and the guide wire developing structure; and a plurality of scales are provided in the scale mark zone, one of the plurality of scales disposed at a farthest end in the scale mark zone is a reference mark point, and one of the plurality of scales farthest from the reference mark point is a last mark point, wherein a distance between the reference mark point and the guide wire developing structure is b, and a distance between the reference mark point and the last mark point is f, $a \leq b + f$.

3. The measuring device according to claim 2, wherein the catheter developing structure is disposed at the distal end portion of the catheter, and b=a.

4. The measuring device according to claim 2, wherein the axial length of the implant corresponding to the minimum specifications is labeled as c, $a \geq b - c$.

5. The measuring device according to claim 4, wherein a distance between the reference mark point and a nearest scale is equal to the axial length corresponding to the implant with the minimum specifications.

6. The measuring device according to claim 5, wherein a distance between any one of the plurality of scales except the reference mark point and the reference mark point is equal to the axial length of the implant with one type of specifications.

7. The measuring device according to any of claims 2-4, wherein the plurality of scales are equally spaced apart.

8. The measuring device according to any of claims 1-6, wherein the catheter developing structure is disposed at the distal end portion of the catheter.

9. The measuring device according to any of claims 1-6, wherein a distance between the proximal end portion of the catheter and the catheter developing structure is labeled as a, and a ranges from 900 mm to 950 mm.

10. The measuring device according to claim 1, wherein the measuring device measures the diseased tissue; the proximal end portion of the catheter is configured to enter into the scale mark zone in response to the guide wire developing structure being aligned with the distal end of the diseased tissue and the catheter developing structure is aligned with the proximal end of the diseased tissue.

Fig. 1

Fig. 2

Fig. 3

Fig. 4

Fig. 5

21

G

W

22

24

201

Fig. 6

G

20

24

23

22

W

Fig. 7

Fig. 8

Fig. 9

B

20

13

22

Fig. 10

Fig. 11

Fig. 12

Fig. 13

Fig. 14

Fig. 15

Fig. 16

E

32

11

4

201

Fig. 17

Fig. 18

## INTERNATIONAL SEARCH REPORT

| International application No. |
|---|
| **PCT/CN2021/131170** |

**A.    CLASSIFICATION OF SUBJECT MATTER**

A61B 5/107(2006.01)i

According to International Patent Classification (IPC) or to both national classification and IPC

**B.      FIELDS SEARCHED**

Minimum documentation searched (classification system followed by classification symbols)

A61B5/-

Documentation searched other than minimum documentation to the extent that such documents are included in the fields searched

Electronic data base consulted during the international search (name of data base and, where practicable, search terms used)

CNPAT, WPI, EPODOC, CNKI: 测量, 导丝, 导管, 鞘管, 套管, 刻度, 刻线, 刻度线, 标尺, 显影, 标识, 标记, 不透射线, 不透辐射, 不透过, 透不过, measur+, catheter, guidewire, mark????, indicia?, tag?, sign?, guage, graduation?, scale? , radiopaque, develop+

**C.      DOCUMENTS CONSIDERED TO BE RELEVANT**

| Category* | Citation of document, with indication, where appropriate, of the relevant passages | Relevant to claim No. |
|---|---|---|
| Y | CN 102209570 A (PNEUMRX, INC.) 05 October 2011 (2011-10-05) description, paragraphs [0186]-[0190] and figure 52 | 1-10 |
| Y | CN 106456938 A (CLEARSTREAM TECH. LTD.) 22 February 2017 (2017-02-22) description, paragraphs [0059]-[0065] and figures 7-11 | 1-10 |
| A | CN 201076667 Y (WU, Jianhua) 25 June 2008 (2008-06-25) entire document | 1-10 |
| A | CN 208990005 U (LINGXIU BIOLOGICAL TECHNOLOGY (SHANGHAI) CO., LTD.) 18 June 2019 (2019-06-18) entire document | 1-10 |
| A | CN 204708809 U (ZHANG, Liping et al.) 21 October 2015 (2015-10-21) entire document | 1-10 |
| A | JP 2007020885 A (ASAHI INTECH K.K. et al.) 01 February 2007 (2007-02-01) entire document | 1-10 |
| A | US 2019000405 A1 (SURGENTEC L.L.C.) 03 January 2019 (2019-01-03) entire document | 1-10 |

☐ Further documents are listed in the continuation of Box C.          ☑ See patent family annex.

| | |
|---|---|
| *      Special categories of cited documents:<br>"A"    document defining the general state of the art which is not considered to be of particular relevance<br>"E"    earlier application or patent but published on or after the international filing date<br>"L"    document which may throw doubts on priority claim(s) or which is cited to establish the publication date of another citation or other special reason (as specified)<br>"O"    document referring to an oral disclosure, use, exhibition or other means<br>"P"    document published prior to the international filing date but later than the priority date claimed | "T"    later document published after the international filing date or priority date and not in conflict with the application but cited to understand the principle or theory underlying the invention<br>"X"    document of particular relevance; the claimed invention cannot be considered novel or cannot be considered to involve an inventive step when the document is taken alone<br>"Y"    document of particular relevance; the claimed invention cannot be considered to involve an inventive step when the document is combined with one or more other such documents, such combination being obvious to a person skilled in the art<br>"&"    document member of the same patent family |

| Date of the actual completion of the international search | Date of mailing of the international search report |
|---|---|
| **13 January 2022** | **27 January 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
Information on patent family members

| International application No. |
| --- |
| **PCT/CN2021/131170** |

| Patent document cited in search report | | | Publication date (day/month/year) | Patent family member(s) | | | Publication date (day/month/year) |
| --- | --- | --- | --- | --- | --- | --- | --- |
| CN | 102209570 | A | 05 October 2011 | CN | 105012058 | A | 04 November 2015 |
| | | | | US | 2010070050 | A1 | 18 March 2010 |
| | | | | US | 9173669 | B2 | 03 November 2015 |
| | | | | WO | 2010030993 | A1 | 18 March 2010 |
| | | | | US | 2016113657 | A1 | 28 April 2016 |
| | | | | US | 10285707 | B2 | 14 May 2019 |
| | | | | DK | 2848208 | T3 | 15 January 2018 |
| | | | | PL | 2848208 | T3 | 30 March 2018 |
| | | | | HK | 1253522 | A1 | 21 June 2019 |
| | | | | US | 2014188246 | A1 | 03 July 2014 |
| | | | | US | 9192403 | B2 | 24 November 2015 |
| | | | | EP | 2848208 | A2 | 18 March 2015 |
| | | | | EP | 2848208 | B1 | 25 October 2017 |
| | | | | US | 2013103059 | A1 | 25 April 2013 |
| | | | | US | 10058331 | B2 | 28 August 2018 |
| | | | | ES | 2654492 | T3 | 13 February 2018 |
| | | | | EP | 3305234 | A1 | 11 April 2018 |
| | | | | HK | 1204251 | A1 | 13 November 2015 |
| | | | | US | 2010100196 | A1 | 22 April 2010 |
| | | | | US | 8632605 | B2 | 21 January 2014 |
| | | | | CN | 102209570 | B | 12 August 2015 |
| | | | | CA | 2737186 | A1 | 18 March 2010 |
| | | | | CA | 2737186 | C | 05 November 2019 |
| | | | | JP | 2015044074 | A | 12 March 2015 |
| | | | | JP | 6016876 | B2 | 26 October 2016 |
| | | | | JP | 2012501813 | A | 26 January 2012 |
| | | | | JP | 5722218 | B2 | 20 May 2015 |
| | | | | HK | 1215853 | A1 | 23 September 2016 |
| | | | | JP | 2016209769 | A | 15 December 2016 |
| | | | | EP | 2341970 | A1 | 13 July 2011 |
| | | | | US | 2019216466 | A1 | 18 July 2019 |
| CN | 106456938 | A | 22 February 2017 | CL | 2016002335 | A1 | 10 March 2017 |
| | | | | WO | 2015153599 | A1 | 08 October 2015 |
| | | | | AU | 2020200172 | A1 | 30 January 2020 |
| | | | | AU | 2020200172 | B2 | 12 November 2020 |
| | | | | AU | 2021200515 | A1 | 25 February 2021 |
| | | | | JP | 2017512587 | A | 25 May 2017 |
| | | | | EP | 3125984 | A1 | 08 February 2017 |
| | | | | EP | 3125984 | B1 | 28 July 2021 |
| | | | | US | 2017021139 | A1 | 26 January 2017 |
| | | | | RU | 2019112030 | A | 06 May 2019 |
| | | | | KR | 20160140657 | A | 07 December 2016 |
| | | | | JP | 2021098132 | A | 01 July 2021 |
| | | | | CA | 2944354 | A1 | 08 October 2015 |
| | | | | CA | 2944354 | C | 03 August 2021 |
| | | | | AU | 2020256411 | A1 | 12 November 2020 |
| | | | | AU | 2015240941 | A1 | 29 September 2016 |
| | | | | AU | 2015240941 | B2 | 10 October 2019 |
| | | | | NZ | 724237 | A | 31 July 2020 |
| | | | | MX | 2016012730 | A | 12 December 2016 |

Form PCT/ISA/210 (patent family annex) (January 2015)

**INTERNATIONAL SEARCH REPORT**
Information on patent family members

International application No.

**PCT/CN2021/131170**

| Patent document cited in search report | | | Publication date (day/month/year) | Patent family member(s) | | | Publication date (day/month/year) |
|---|---|---|---|---|---|---|---|
| | | | | RU | 2016141624 | A | 07 May 2018 |
| | | | | RU | 2686297 | C2 | 24 April 2019 |
| CN | 201076667 | Y | 25 June 2008 | None | | | |
| CN | 208990005 | U | 18 June 2019 | None | | | |
| CN | 204708809 | U | 21 October 2015 | None | | | |
| JP | 2007020885 | A | 01 February 2007 | JP | 4707140 | B2 | 22 June 2011 |
| US | 2019000405 | A1 | 03 January 2019 | US | 10842448 | B2 | 24 November 2020 |

Form PCT/ISA/210 (patent family annex) (January 2015)